# EUROPEAN PATENT APPLICATION

(11) **EP 4 483 929 A1**
(43) Date of publication of application: **01.01.2025**
(21) Application number: 23857496.6
(22) Date of filing: 02.06.2023
(51) Int. Cl.: A61M 15/00, A24F 40/20, A24F 40/42, A24F 40/40

(54) **INHALER**

(30) Priority: 25.08.2022 KR 20220106750
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SHIN, Jun Won, Daejeon 34128 (KR)
(74) Representative: Ter Meer Steinmeister & Partner
(86) International application number: PCT/KR2023/007660
(87) International publication number: WO 2024/043457

(57) **Abstract**

An inhaler for inhaling a functional substance according to various embodiments may include a first holder, a second holder rotatably connected to the first holder, a piercing member protruding toward an internal space of the second holder, and a functional substance accommodation member disposed in the internal space of the second holder and configured to accommodate the functional substance, wherein at least a portion of the functional substance accommodation member may be open by the piercing member according to a rotation of the second holder.

## Description

### TECHNICAL FIELD

The following embodiments relate to an inhaler.

### BACKGROUND ART

Research has been conducted on an inhaler that may deliver a target substance directly to the lungs of a user. For example, Korean Patent Publication No. 10-2016-0065204 discloses a dry powder inhaler.

### DISCLOSURE OF THE INVENTION

### TECHNICAL GOALS

An inhaler according to various embodiments may induce a vortex behavior of functional substance powder.

An inhaler according to various embodiments may induce smooth discharge of functional substance powder.

### TECHNICAL SOLUTIONS

An inhaler according to various embodiments may include a first holder, a second holder rotatably connected to the first holder, at least two piercing members protruding toward an internal space of the second holder, and a functional substance accommodation member disposed in the internal space of the second holder and configured to accommodate the functional substance, wherein at least a portion of the functional substance accommodation member may be open by the piercing member according to a rotation of the second holder.

According to an embodiment, the piercing members may be disposed spaced apart from each other along the rotation axis of the second holder. The piercing members may be disposed on opposite sides of the rotation axis of the second holder.

According to an embodiment, the piercing members may be disposed protruding in a direction perpendicular to a rotation axis of the second holder.

According to an embodiment, the second holder may be rotatable relative to the first holder between a first rotation position and a second rotation position, and a rotation angle of the second holder at the first rotation position may be less than a rotation angle of the second holder at the second rotation position. A rotation angle marker for indicating a rotation angle of the second holder may be provided in one of the first holder or the second holder.

According to an embodiment, an internal space of the first holder and internal space of the second holder may be configured in cylindrical shapes connected to each other, and the functional substance accommodation member may be configured to be insertable into the internal space of the first holder and the internal space of the second holder.

According to an embodiment, the functional substance accommodation member may include a stick configured to be insertable into the internal space of the first holder and the internal space of the second holder and a capsule disposed in an inside of the stick and configured to accommodate the functional substance.

According to an embodiment, a diameter of the internal space of the second holder may be formed to be greater than a diameter of the internal space of the first holder.

According to an embodiment, the piercing member may be configured to be movable between a first piercing position and a second piercing position, and the second piercing position may be configured to protrude further toward the rotation axis of the second holder than the first piercing position.

According to an embodiment, the functional substance accommodation member may include a capsule accommodating the functional substance, and a portion of the capsule may be fixed to the first holder so that the capsule may be rotatable relative to the second holder.

According to an embodiment, the piercing member may be disposed in the second holder in a state of penetrating at least a portion of the capsule.

### EFFECTS OF THE INVENTION

According to various embodiments, a vortex behavior of functional substance powder may be induced.

According to various embodiments, smooth discharge of functional substance powder may be performed by internal airflow introduced into an inhaler penetrating a center of a functional substance accommodation member.

The effects of an inhaler according to various embodiments are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 is a diagram illustrating an inhaler according to various embodiments.
FIGs. 2A to 2D are diagrams illustrating an operating state of an inhaler of various embodiments.
FIG. 3 is a diagram illustrating an inhaler according to various embodiments.
FIGs. 4A and 4B are diagrams illustrating an operating state of an inhaler of various embodiments.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

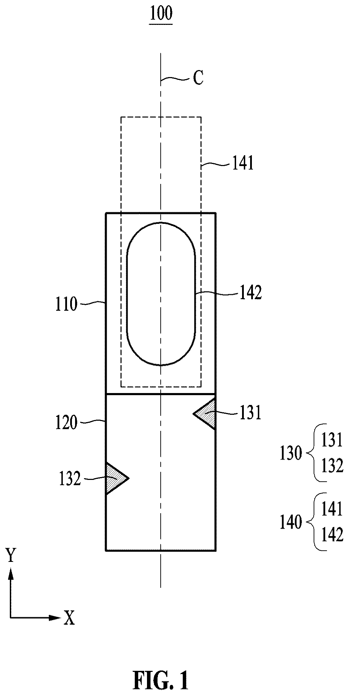

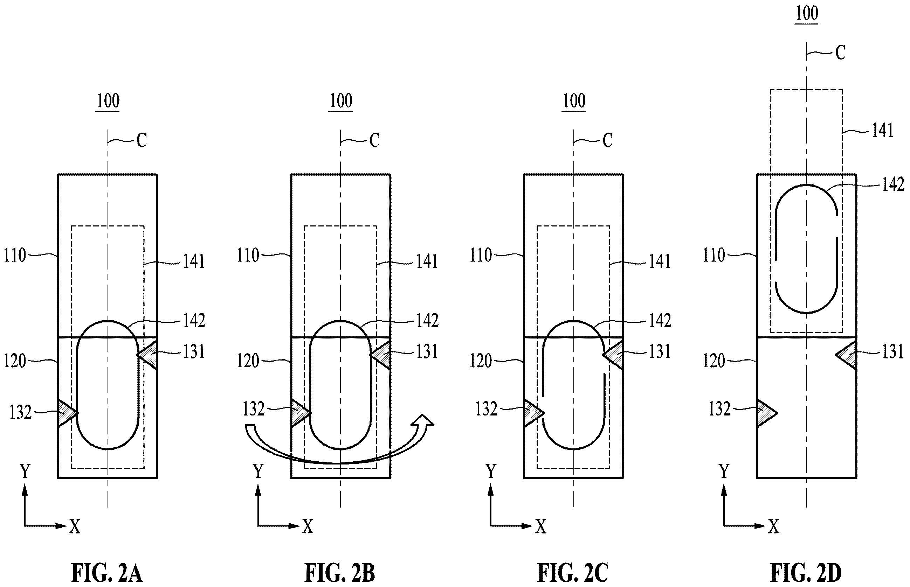

FIG. 1 is a diagram illustrating an inhaler 100 according to various embodiments, and FIGs. 2A to 2D are diagrams illustrating an operating state of the inhaler 100 according to various embodiments.

The inhaler 100 according to an embodiment may deliver a functional substance to a user. For example, the inhaler 100 according to an embodiment may be composed of an inhaler that delivers nicotine to the lungs of a user in a form of an aerosol.

Referring to FIG. 1, the inhaler 100 according to an embodiment may include a first holder 110, a second holder 120, a piercing member 130, and a functional substance accommodation member 140.

In an embodiment, the first holder 110 and the second holder 120 may be rotatably connected. For example, the first holder 110 may be configured in a cylindrical shape with an internal space, and the second holder 120 may also be configured in a cylindrical shape with an internal space. One end portion of the first holder 110 (e.g., an end portion in a -Y direction of the first holder 110 of FIG. 1) may be rotatably coupled to one end of the second holder 120 (e.g., an end portion in a +Y direction of the second holder 120 of FIG. 1).

The internal space of the first holder 110 and the internal space of the second holder 120 may be connected and communicate with each other, and the internal spaces may be formed to a size into which the functional substance accommodation member 140, which is described below, may be inserted.

In an embodiment, the piercing member 130 may include a first piercing member 131 and a second piercing member 132. One side of the first piercing member 131 may be fixed to an inside of the second holder 120, and the other side of the first piercing member 131 may be configured as a cutting edge protruding toward a rotation axis C of the second holder 120. One side of the second piercing member 132 may be fixed to the inside of the second holder 120, and the other side of the second piercing member 132 may be configured as a cutting edge protruding toward the rotation axis C of the second holder 120.

The first piercing member 131 and the second piercing member 132 may be disposed to be spaced apart in a direction parallel to the rotation axis C. For example, the first piercing member 131 and the second piercing member 132 may be disposed to be spaced apart from each other in a ±Y direction of FIG. 1. In addition, the first piercing member 131 and the second piercing member 132 may be disposed to face each other about the rotation axis C. For example, the first piercing member 131 and the second piercing member 132 may be disposed to be spaced apart from each other in a ±X direction of FIG. 1 with respect to the central axis C.

In an embodiment, the functional substance accommodation member 140 may include a stick 141 and a capsule 142. For example, the stick 141 may be configured in a cylindrical shape that may be inserted into the internal space of the first holder 110 and the internal space of the second holder 120, and the capsule 142 may be disposed in an inside of the stick 141.

An area of the functional substance accommodation member 140 in which the capsule 142 may be disposed may be set so that the capsule 142 may be in a position facing the first piercing member 131 or the second piercing member 132 when the functional substance accommodation member 140 is inserted in the internal space of the second holder 120.

In an example, the capsule 142 may accommodate a functional substance. The functional substance may include, for example, at least one of nicotine, theanine, caffeine, taurine, or a mixture thereof. The functional substance may be in a form of fine granules or dry powder.

In an example, an outer shell of the capsule 141 may be made of a material that may be pierced or cut by the first piercing member 131 or the second piercing member 132.

When the functional substance accommodation member 140 is inserted into the second holder 120, the functional substance accommodation member 140 may not be fixed to the second holder 120, so that the functional substance accommodation member 140 and the second holder 120 may freely rotate relative to each other. For example, a diameter of the internal space of the second holder 120 may be formed greater than a diameter of the internal space of the first holder 110. In this case, a portion of the functional substance accommodation member 140 may be fixed to the first holder 110 in an interference fit manner and may rotate together, while another portion (e.g., an area of the capsule 143) of the functional substance accommodation member 140 may not be fixed to the second holder 120, so the second holder 120 may rotate around a circumference of the functional substance accommodation member 140.

In an embodiment, the first piercing member 131 and the second piercing member 132 may move between a first piercing position and a second piercing position, and the second piercing position may protrude further toward the rotation axis C of the second holder 120 than the first piercing position. Here, a horizontal distance (e.g., a ±X direction distance of FIG. 1) between the first piercing member 131 and the second piercing member 132 at the first piercing position may be set to be greater than an outer diameter of the functional substance accommodation member 140, and the horizontal distance (e.g., the ±X direction distance of FIG. 1) between the first piercing member 131 and the second piercing member 132 at the second piercing position may be set to be less than an outer diameter of the capsule 142.

For example, before the functional substance accommodation member 140 is inserted into the internal space of the second holder 120, the first piercing member 131 and the second piercing member 132 may be at the first piercing position. Thereafter, the functional substance accommodation member 140 may be inserted into the internal space of the second holder 120 without being disturbed by the first piercing member 131 or the second piercing member 132. In a state in which the functional substance accommodation member 140 is inserted into the internal space of the second holder 120, the first piercing member 131 and the second piercing member 132 may be at the second piercing position. At the second piercing position, the first piercing member 131 or the second piercing member 132 may penetrate the capsule 142 so that at least a portion of the capsule 142 may be open and the functional substance in the capsule 142 may be exposed to an outside of the capsule 142.

Alternatively, the first piercing member 131 and the second piercing member 132 may be at the second piercing position both before and after the functional substance accommodation member 140 is inserted into the internal space of the second holder 120. Here, when the functional substance accommodation member 140 is inserted into the second holder 120, an open area (a longitudinal slit) may additionally be formed in the functional substance accommodation member 140 in a direction (e.g., a ±Y direction of FIG. 1) parallel to the rotation axis C.

In an embodiment, the second holder 120 may be rotatable relative to the first holder 110 between a first rotation position and a second rotation position, and a rotated angle of the second holder 120 with respect to an initial position at the first rotation position may be less than a rotated angle of the second holder 120 with respect to an initial position at the second rotation position.

For example, when the second holder 120 rotates in a state in which the piercing member 130 penetrates the capsule 142, an open area in a circumferential direction may be formed in the capsule 142, and the functional substance may be discharged through the open area. Here, since the open area at the second rotation position may be greater than the open area at the first rotation position, the user may easily adjust a transfer amount of the functional substance by adjusting the open area to suit a preference of the user.

In an example, a rotation angle marker for indicating a rotation angle of the second holder 120 may be provided in one of the first holder 110 or the second holder 120. For example, the rotation angle marker may indicate the first rotation position or the second rotation position.

Hereinafter, the operating state of the inhaler 100 according to various embodiments is described with reference to FIGs. 2A to 2D.

Referring to FIG. 2A, the functional substance accommodation member 140 may be inserted into the internal space of the first holder 110 and the internal space of the second holder 120. Here, the first piercing member 131 or the second piercing member 132 may be moved from the first piercing position to the second piercing position, and the capsule 142 may be pierced by the first piercing member 131 or the second piercing member 132.

Referring to FIG. 2B, the second holder 120 may rotate relative to the first holder 110. Here, since the stick 141 may be fixed to the first holder 110 and may not be fixed to the second holder 120, with a rotation of the second holder 120, the first piercing member 131 or the second piercing member 132 may cut a portion of the capsule 142, thereby forming an open area in the capsule 142 in the circumferential direction.

Referring to FIG. 2C, the rotation of the second holder 120 may stop at the first rotation angle or the second rotation angle. Here, the user may set the first rotation angle or the second rotation angle according to a desired degree of transfer of the functional substance. When the rotation of the second holder 120 is completed, the open area may be formed in a slit shape in the capsule 142, and a slit by the first piercing member 131 and a slit by the second piercing member 132 may be formed, respectively. Since the slits may be formed to face each other and to be spaced apart along a direction parallel to the central axis C, airflow passing through the slits may pass through a central area of the capsule 142.

Referring to FIG. 2D, the functional substance accommodation member 140 may be separated from the second holder 120. Here, the functional substance accommodation member 140 may be separated from the first holder 110, but the functional substance may be delivered while the functional substance accommodation member 140 is inserted in the first holder 110. In this case, separation of the functional substance may effectively be prevented compared to a case in which the functional substance accommodation member 140 is completely separated from the first holder 110, and thus, unnecessary leakage of the functional substance may be prevented.

When the user bites one end of the functional substance accommodation member 140 and makes an inhaling motion while the slit is formed in the capsule 142, external air may flow into the internal space of the first holder 110 through an airflow hole that may be formed in the first holder 110 or the second holder 120, and the air may flow into the capsule 142 through the slit formed by the second piercing member 132. Subsequently, the air may escape through the slit formed by the first piercing member 131 and reach the user, in which case the functional substance in the capsule 142 may also be transferred to the user. Due to eccentric positions of the slits, the air may penetrate a central part of the capsule 142 and, at the same time, a vortex behavior of the functional substance may be induced. The vortex behavior may induce smooth movement of the functional substance, and accordingly, discharge of the functional substance may be performed more smoothly.

In an example, when the capsule 142 is disposed in the stick 141 in an unfixed state, the rotation of the capsule 142 may be induced by eccentric air movement (airflow) generated due to an eccentric position of the slit. Through this, the discharge of the functional substance may be performed more smoothly.

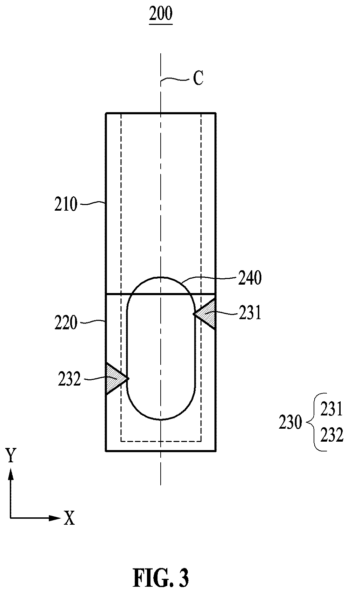

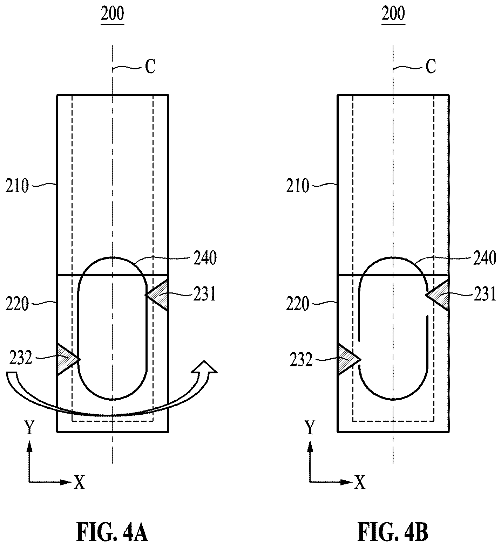

FIG. 3 is a diagram illustrating an inhaler 200 according to various embodiments, and FIGs. 4A and 4B are diagrams illustrating an operating state of the inhaler 200 according to various embodiments.

Referring to FIG. 3, the inhaler 200 according to an embodiment may include a first holder 210, a second holder 220, a piercing member 230, and a functional substance accommodation member 240. Structures and functions of the first holder 210, the second holder 220, and the piercing member 230 may be identical or similar to those of the first holder 110, the second holder 120, and the piercing member 130 described above, and a description thereof is thus omitted for simplicity.

In an embodiment, the functional substance accommodation member 240 may be composed of a capsule accommodating a functional substance, and a portion of the capsule may be fixed to a side of the first holder 210 so that the capsule may rotate relative to the second holder 220. In an example, the capsule may accommodate a functional substance. The functional substance may include, for example, at least one of nicotine, theanine, caffeine, taurine, or a mixture thereof. The functional substance may be in a form of fine granules or dry powder. In an example, an outer shell of the capsule may be made of a material that may be pierced or cut by the first piercing member 231 or the second piercing member 232.

The functional substance accommodation member 240 may be disposed being inserted into an internal space of the second holder 220. Here, the functional substance accommodation member 240 may be disposed in an internal area of the second holder 220, adjacent to the first piercing member 231 and the second piercing member 232.

In an embodiment, the first piercing member 231 and the second piercing member 232 may move between a third piercing position and a fourth piercing position, and the fourth piercing position may protrude further toward a rotation axis C of the second holder 220 than the third piercing position. Here, a horizontal distance (e.g., a ±X direction distance of FIG. 3) between the first piercing member 231 and the second piercing member 232 at the third piercing position may be set to be greater than an outer diameter of the functional substance accommodation member 240, and the horizontal distance (e.g., the ±X direction distance of FIG. 3) between the first piercing member 231 and the second piercing member 232 at the fourth piercing position may be set to be less than an outer diameter of the functional substance accommodation member 240.

For example, before using the inhaler 200 according to an embodiment, the first piercing member 231 and the second piercing member 232 may be at the third piercing position. When a user intends to use the inhaler 200 according to an embodiment, the first piercing member 231 and the second piercing member 232 may move to the fourth piercing position. At the fourth piercing position, the first piercing member 231 or the second piercing member 232 may penetrate the functional substance accommodation member 240 so that at least a portion of the functional substance accommodation member 240 may be open and the functional substance in the functional substance accommodation member 240 may be exposed to an outside of the functional substance accommodation member 240.

Alternatively, at least one of the first piercing member 231 or the second piercing member 232 may be disposed in the second holder 220 in a state of penetrating at least a portion of the functional substance accommodation member 240.

In an embodiment, the second holder 220 may be rotatable relative to the first holder 210 between a third rotation position and a fourth rotation position, and a rotated angle of the second holder 220 with respect to an initial position at the third rotation position may be less than a rotated angle of the second holder 220 with respect to an initial position at the fourth rotational position.

Hereinafter, the operating state of the inhaler 200 according to an embodiment is described with reference to FIGs. 4A and 4B.

Referring to FIG. 4A, the second holder 220 may rotate relative to the first holder 210 in a state in which the functional substance accommodation member 240 is penetrated by the first piercing member 231 or the second piercing member 232.

Here, with a rotation of the second holder 220, the first piercing member 231 or the second piercing member 232 may cut a portion of the functional substance accommodation member 240, thereby forming an open area in the functional substance accommodation member 240 in a circumferential direction.

Referring to FIG. 4B, the rotation of the second holder 220 may stop at the third rotation angle or the fourth rotation angle. Here, the user may set the third rotation angle or the fourth rotation angle according to a desired degree of transfer of the functional substance. When the rotation of the second holder 220 is completed, the open area may be formed in a slit shape in the circumferential direction in the functional substance accommodation member 240, and a slit by the first piercing member 231 and a slit by the second piercing member 232 may be formed, respectively. Since the slits may be formed to face each other and to be spaced apart along a direction parallel to the central axis C, airflow passing through the slits may pass through a central area of the functional substance accommodation member 240.

When the user bites one end of the first holder 210 and makes an inhaling motion while the slit is formed in the functional substance accommodation member 240, external air may flow into the internal space of the second holder 220 through an airflow hole that may be formed in the first holder 210 or the second holder 220, and the air may flow into the functional substance accommodation member 240 through the slit formed by the second piercing member 232. Subsequently, the air may escape through the slit formed by the first piercing member 231 and reach the user, in which case the functional substance in the functional substance accommodation member 240 may also be transferred to the user. Due to eccentric positions of the slits, the air may penetrate a central part of the functional substance accommodation member 240 and, at the same time, a vortex behavior of the functional substance may be induced. The vortex behavior may induce smooth movement of the functional substance, and accordingly, discharge of the functional substance may be performed more smoothly.

The description of the above-described embodiments is only an example, and one of ordinary skill in the art will understand that various modifications and equivalents may be made therefrom. Therefore, the scope of protection of the present disclosure should be defined by the appended claims, and all differences within the equivalent scope of what is stated in the claims should be construed as being included in the scope of protection defined by the claims.

## Claims

1. An inhaler for inhaling a functional substance, the inhaler comprising:
a first holder;
a second holder rotatably connected to the first holder;
a piercing member protruding toward an internal space of the second holder; and
a functional substance accommodation member disposed in the internal space of the second holder and configured to accommodate the functional substance,
wherein at least a portion of the functional substance accommodation member is open by the piercing member according to a rotation of the second holder.

2. The inhaler of claim 1, wherein the piercing member comprises:
a first piercing member; and
a second piercing member disposed spaced apart from the first piercing member in a direction parallel to a rotation axis of the second holder.

3. The inhaler of claim 2, wherein the first piercing member and the second piercing member are disposed to face each other about the rotation axis of the second holder.

4. The inhaler of claim 1, wherein a cutting edge of the first piercing member and a cutting edge of the second piercing member are directed toward a rotation axis of the second holder.

5. The inhaler of claim 1, wherein
the second holder is rotatable relative to the first holder between a first rotation position and a second rotation position; and
a rotation angle of the second holder at the first rotation position is less than a rotation angle of the second holder at the second rotation position.

6. The inhaler of claim 5, wherein a rotation angle marker for indicating a rotation angle of the second holder is provided in one of the first holder or the second holder.

7. The inhaler of one of claim 1 to claim 6, wherein
an internal space of the first holder and the internal space of the second holder are configured in cylindrical shapes connected to each other, and
the functional substance accommodation member is configured to be insertable into the internal space of the first holder and the internal space of the second holder.

8. The inhaler of claim 7, wherein the functional substance accommodation member comprises:
a stick configured to be insertable into the internal space of the first holder and
the internal space of the second holder; and
a capsule disposed in an inside of the stick and configured to accommodate the functional substance.

9. The inhaler of claim 8, wherein a diameter of the internal space of the second holder is formed to be greater than a diameter of the internal space of the first holder.

10. The inhaler of claim 2 or claim 3, wherein
the first piercing member and the second piercing member are configured to be movable between a first piercing position and a second piercing position, and
the second piercing position is configured to protrude further toward the rotation axis of the second holder than the first piercing position.

11. The inhaler of claim 1, wherein
the functional substance accommodation member comprises a capsule accommodating the functional substance, and
a portion of the capsule is fixed to the first holder so that the capsule is rotatable relative to the second holder.

12. The inhaler of claim 11, wherein the piercing member is disposed in the second holder in a state of penetrating at least a portion of the capsule.
